# EUROPÄISCHE PATENTANMELDUNG

(11) **EP 0 615 748 A1**
(43) Veröffentlichungstag der Anmeldung: **21.09.1994**
(21) Anmeldenummer: 94102418.4
(22) Anmeldetag: 17.02.1994
(51) Int. Cl.: A61K 9/16, A61K 7/00

(54) **Vorzugsweise in Form von Mikrosphärulen vorliegende galenische Matrices**

(30) Priorität: 16.03.1993 DE 4308282
(71) Anmelder: Beiersdorf Aktiengesellschaft, D-20245 Hamburg (DE)
(72) Erfinder: Heinze, Friedrich, Dr., D-60318 Frankfurt (DE); Clasen, Martina, D-20253 Hamburg (DE)

(57) **Zusammenfassung**

Zusammensetzungen aus
(a) einem oder mehreren Fettalkoholen, gewählt aus der Gruppe der verzweigten oder unverzweigten Alkylalkohole mit 16 - 22 C-Atomen,
(b) einem oder mehreren Wachsestern,
(c) gegebenenfalls hochdispersem Siliciumdioxid,
(d) gegebenenfalls einen oder mehrere kosmetische und/oder pharmazeutische Wirkstoffe enthaltend,
(e) sowie gegebenenfalls übliche pharmazeutische bzw. kosmetische Hilfs- und/oder Zusatzstoffen enthaltend.

## Beschreibung

Die vorliegende Erfindung betrifft kosmetische und pharmazeutische Matrices zur Darreichung kosmetischer bzw. pharmazeutischer Wirkstoffe. Insbesondere betrifft die vorliegende Erfindung Matrices, welche zum Schutze gegen chemische Zersetzung empfindlicher Wirkstoffe geeignet sind.

Insbesondere betrifft die Erfindung Wirkstoffträgermatrices zur gezielten und/oder verzögerten Freisetzung kosmetischer oder pharmazeutischer Wirkstoffe. Weiterhin betrifft die Erfindung Verfahren zur Herstellung von Wirkstoffträgermatrices sowie deren Verwendung für kosmetische und pharmazeutische Zwecke.

Schließlich betrifft die Erfindung kosmetische bzw. pharmazeutische Zubereitungen, mit einem Gehalt an kosmetischen bzw. pharmazeutischen Matrices.

Die Erfindung betrifft bevorzugt die topische Applikation.

Wirkstoffträgersysteme für kosmetische oder pharmazeutische Verwendungen sind an sich gut bekannt und weit verbreitet. Beispiele für partikuläre Systeme sind Mikrokapseln, Nanokapseln und Liposomen bzw. Niosomen. Das einfachste Wirkstoffträgersystem ist die kosmetische oder pharmazeutische Emulsion, bei welcher ein Wirkstoff in die dispergierte Phase eingearbeitet ist.

Ein grobes Unterscheidungsraster für diese Darreichungsformen ist ihre Größe. Die Größe der Mikrokapseln, wie auch die der dispergierten Emulsionströpfchen, liegt im Mikrometerbereich. Die Größe der Nanokapseln liegt im Nanometerbereich. Liposomen und Niosomen nehmen Größen einiger Nanometer bis einiger Mikrometer ein. Niosomen stellen Varianten der Liposomen dar, welche sich durch eine besondere Membranzusammensetzung auszeichnen.

Wirkstoffträger werden einesteils eingesetzt, um den oder die Wirkstoffe vor Umwelteinflüssen wie Oxidation oder Licht zu schützen. Ein anderer Bestimmungszweck kann sein, mit Hilfe des betreffenden Trägersystems den oder die Wirkstoffe gezielt am Anwendungsort freizusetzen. So wird beispielsweise angestrebt, mit Hilfe von kosmetischen Zubereitungen auf der Basis von Liposomen Wirkstoffe nicht auf der Haut verweilen zu lassen, sondern sie durch Diffusion durch die oberste in die tieferen Hautschichten zu transportieren. Dort soll sich die Wandung der Liposomen auflösen und die Wirkstoffe freisetzen. Würde der Wirkstoff ohne ein solches System auf die Haut aufgetragen, so würde der Wirkstoff nutzlos oder doch größtenteils ungenutzt auf der Haut verbleiben, oder er würde nicht optimal oder exakt am eigentlichen Wirkort zur Wirkung gelangen.

Mikrokapseln sind rieselfähige Pulver oder Puder mit Partikeldurchmessern im Bereich von ca. 1 µm - 1.000 µm. Sie werden unter Verwendung verschiedener Umhüllungsprozesse feinverteilter fester, flüssiger, sogar gasförmiger Stoffe hergestellt. Als Hüll- bzw. Wandmaterial werden üblicherweise Polymere verwendet. Grundsätzlich bestehen Mikrokapseln also aus zwei disparaten Bereichen, dem Kernbereich und dem Hüllbereich.

Für die Mikroverkapselung geeignete Herstellungsverfahren sind Phasentrennverfahren (einfache und komplexe Koazervation), Grenzflächenpolymerisationsverfahren (Polykondensation bzw. Polyaddition aus Dispersionen) und mechanisch-physikalische Verfahren (Wirbelschichtverfahren, Sprühtrocknung).

Grundsätzlicher Nachteil der herkömmlichen Mikroverkapselung ist der relativ hohe Herstellungsaufwand.

Mikrosphärulen sind den Mikrokapseln verwandte Systeme, bei denen eine exakte Trennung in Kern- und Hüllbereich nicht vorliegt. In ihrer Größe von ca. 1 µm - 1.000 µm entsprechen Mikrosphärulen allerdings den Mikrokapseln. In Mikrosphärulen liegt der eingebettete Wirkstoff in fester, d.h. dispergierter, oder gelöster Form in die Trägermatrix inkorporiert vor. Mikrosphärulen sind also eine Sonderform von Mikrokapseln.

Unter kosmetischer Hautpflege ist in erster Linie zu verstehen, daß die natürliche Funktion der Haut als Barriere gegen Umwelteinflüsse (z.B. Schmutz, Chemikalien, Mikroorganismen) und gegen den Verlust von körpereigenen Stoffen (z.B. Wasser, natürliche Fette, Elektrolyte) gestärkt oder wiederhergestellt wird.

Wird diese Funktion gestört, kann es zu verstärkter Resorption toxischer oder allergener Stoffe oder zum Befall von Mikroorganismen und als Folge zu toxischen, irritativen oder allergischen Hautreaktionen kommen.

Ziel der Hautpflege ist es ferner, den durch tägliche Waschen verursachten Fett- und Wasserverlust der Haut auszugleichen. Dies ist gerade dann wichtig, wenn das natürliche Regenerationsvermögen nicht ausreicht. Außerdem sollen Hautpflegeprodukte vor Umwelteinflüssen, insbesondere vor Sonne und Wind, schützen und die Hautalterung verzögern. Dies ist allerdings oft nur durch Einsatz kosmetisch aktiver Wirkstoffe zu bewerkstelligen. Ziel der Entwicklung kosmetischer Zubereitungen ist es dann, die Wirkstoffe stabil in die Formulierungen einzuarbeiten und dort über längere Zeit stabil aufrechtzuerhalten.

Kosmetische Wirkstoffe aber sind häufig vor allem in höherer Wirkkonzentration instabil oder aber inkompatibel zu anderen Bestandteilen der Zubereitungen.

Medizinische topische Zusammensetzungen enthalten als pharmazeutische Wirkstoffe in der Regel ein oder mehrere Medikamente in wirksamer Konzentration. Der Einfachheit halber wird zur sauberen Unterscheidung zwischen kosmetischer und medizinischer Anwendung und entsprechenden Produkten auf die gesetzlichen Bestimmungen der Bundesrepublik Deutschland verwiesen (z.B. Kosmetikverordnung, Lebensmittel- und Arzneimittelgesetz).

Die Zubereitungen des Standes der Technik weisen jedoch einige Nachteile auf: Durch viele der betreffenden Darreichungsformen ist die chemische Stabilisierung mancher oxidationsempfindlicher, lichtempfindlicher oder hydrolyseempfindlicher Stoffen nicht gewährleistet. Sollen pharmazeutische Wirkstoffe zur Anwendung gebracht werden, ist dieser Nachteil natürlich besonders bedenklich. So ist im Falle von in Emulsionen gelösten Wirkstoffen mit chemischen Umwandlungen der Wirkstoffe oder Reaktionen verschiedener Wirkstoffe untereinander oder Reaktionen der Wirkstoffe mit anderen Stoffen, z.B: Hilfs- oder Zusatzstoffen zu rechnen.

Bei kosmetischer Anwendung ist zu beklagen, daß insbesondere die Fixierung leichtflüchtiger Substanzen, z.B. etherischer Öle, Aromen, Parfums meist unzureichend ist. Es trifft umgekehrt auch häufig zu, daß kosmetische Wirkstoffe charakteristische oder gar unangenehme Eigengerüche aufweisen, welche ohne Kaschierung kosmetisch nicht akzeptabel sind.

Aber auch der Nachteil, daß auch viele kosmetische Wirkstoffe instabil sind und gegen Denaturierung geschützt werden müssen, läßt sich durch herkömmliche Wirkstoffträgersysteme nur in begrenztem Maße bewerkstelligen.

Solche in diesem Sinne instabilen oder zu stabilisierenden Substanzen sind beispielsweise Acetylsalicylsäure, Atropin, Azulen, Hydrocortison und dessen Derivate, z.B. Hydrocortison-17-valerat, Vitamine, z.B. Vitamine der B- und D-Reihe, sehr günstig das Vitamin B₁, das Vitamin B₁₂, das Vitamin D₃, aber auch die Vitamine A, E, K, Acorbinsäure und ihre Derivate, Bisabolol, ungesättigte Fettsäuren, namentlich die essentiellen Fettsäuren (oft auch Vitamin F genannt), insbesondere die gamma-Linolensäure, Ölsäure, Eicosapentaensäure, Docosahexaensäure und ähnliche, ferner natürliche und synthetische Ceramide und ceramidähnliche Verbindungen, weiterhin solche Wirkstoffe wie Chloramphenicol, Prostaglandine, Thymol, Campher, Extrakte oder andere Produkte tierischer und pflanzlicher Herkunft, z.B. Fischöle, Nachtkerzenöl, Borretschöl, Johannisbeersamenöl, Lebertran und so weiter.

Auch die Freisetzungscharakteristiken der Zubereitungen des Standes der Technik sind häufig ungenügend. So kann es geschehen, daß manche Matrices einen Wirkstoff in einem einzigen Schub abgeben, andere wiederum geben den eingearbeiteten Stoff so langsam ab, daß eine kosmetische oder therapeutische Wirkung nicht zustandekommt.

Aus der Firmenschrift von Dow Corning "Offene Fragen zur Verarbeitung von Polytrap R", unter dem Kapitel "Acrylates Copolymer: A Technique for Entrapping Cosmetic Actives" von W.L.Klein und A.J.DiSapio, Dow Corning, Montgomery, New York, sind Zusammensetzungen bekannt, in welchen eine Acrylatmatrix als Trägermatrix für Lidschatten und Rouge sowie als Absorptionsmatrix eines Hautfettadsorbers dient.

Diese Zusammensetzungen haben jedoch den Nachteil, daß die Matrices beim Auftragen auf die Haut zu leicht zerbröseln. Die erwünschte gleichmäßige Freigabe eines Wirkstoffes kann auf diese Weise nicht bewirkt werden.

Die DE-OS 41 27 665 beispielsweise beschreibt galenische Matrices, welche Zusammensetzungen aus
(a) Mikrokristalliner Cellulose,
(b) hochdispersem Siliciumdioxid,
(c) wenigstens einen lipophilen Bestandteil, gewählt aus der Gruppe der Öle, Fette und Wachse
(d) gegebenenfalls Acrylatpolymeren und/oder -copolymeren
darstellen.

Obwohl die in dieser Schrift genannten galenischen Matrices über hervorragende kosmetische bzw. pharmazeutische Eigenschaften verfügen, liegt ihre Stärke jedoch darin, daß ihre positiven Eigenschaften vorwiegend bei Partikeldurchmessern der Größenordnung von 0,5 - 5 mm zur Geltung kommen.

Alle bekannten feinpartikulären Trägersysteme weisen also mindestens einen der folgenden Nachteile auf:
Die Partikel sind schlecht verteilbar, sie sind porös und überdies in den meisten Fallen von sehr unregelmäßiger Form und keineswegs rund.

Die Partikel sind häufig sehr groß, d.h. > 400 µm, auf der Haut daher als Partikel spürbar und nicht topisch einwandfrei applizierbar.

Die Partikelgrößenverteilung ist äußerst inhomogen. Dadurch ist eine gezielte Steuerbarkeit der Wirkstofffreisetzung nicht gewährleistet.

Die Partikel stabilisieren Wirkstoffe wie z.B. ungesättigte Fettsäuren bzw. Nachtkerzenöl, wenn überhaupt, nur unzureichend.

Die Partikel sind nur im Labormaßstab erhältlich, aber nicht großtechnisch herstellbar.

Die Partikel sind nur so aufwendig herstellbar, daß ihr Einsatz in zur Wirkung erforderlichen Konzentration oft wirtschaftlich nicht zu vertreten ist.

Die Partikel, namentlich echte Mikrokapseln, können nur in wäßrigen Systemen hergestellt, gelagert bzw. in den Handel gebracht werden. Daher müssen solchen Systemen stets Konservierungsmittel zugegeben werden. Solche aber sind oft unerwünscht.

Die Partikel sind nur in ungenügender Weise verteilbar und/oder lagerstabil. Ihre in den Endformulierungen eigentlich angestrebten Eigenschaften sind somit nicht erreichbar.

Aufgabe der vorliegenden Erfindung war also, Mittel zur Verfügung zu stellen, welche nicht mit den Mängeln des Standes der Technik behaftet sind.

Insbesondere sollten Trägermatrices zur Verfügung gestellt werden, welche nicht nur über günstige Freigabecharakteristika für kosmetische und pharmazeutische Wirkstoffe verfügen, sondern diese auch während der Lagerung und im Endprodukt stabilisieren. Verhindert werden soll dabei unter anderem, daß der Gehalt an Wirkstoffen bei der Lagerung gemindert wird, daß sich diese Wirkstoffe bei Lagerung zersetzen, also beispielsweise oxidieren, oder unangenehme Eigengerüche freigesetzt werden.

Es hat sich überraschend herausgestellt, und darin liegt die Lösung der Aufgabe begründet, daß Zusammensetzungen aus
(a) einem oder mehreren Fettalkoholen, gewählt aus der Gruppe der verzweigten oder unverzweigten Alkylalkohole mit 16 - 22 C-Atomen,
(b) einem oder mehreren Wachsestern,
(c) gegebenenfalls hochdispersem Siliciumdioxid,
(d) gegebenenfalls einen oder mehrere kosmetische und/oder pharmazeutische Wirkstoffe enthaltend,
(e) sowie gegebenenfalls übliche pharmazeutische bzw. kosmetische Hilfs- und/oder Zusatzstoffen enthaltend,
hervorragende Galenische Matrices darstellen, die die Probleme des Standes der Technik lösen und den Zubereitungen des Standes der Technik überlegen sind.

Vorteilhafte erfindungsgemäße Fettalkohole sind beispielsweise Cetylalkohol, Stearylalkohol, Cetyl-/Stearylalkohol und Behenylalkohol.

Vorteilhafte Wachsester im Sinne der hiermit vorgelegten Lehre zum technischen Handeln sind Ester höhermolekularer wasserunlöslicher Fettalkohole und Fettsäuren. Bevorzugt sind solche Wachsester, bei denen die Anzahl der C-Atome größer ist als etwa 22, und höhermolekularer Fettsäuren, bei welchen die Anzahl der C-Atome größer ist als etwa 14.

Als besonders vorteilhaft hat sich das Cetylpalmitat erwiesen.

Das hochdisperse Siliciumdioxid wird erfindungsgemäß bevorzugt gewählt aus der Gruppe der hochreinen, röntgenamorphen Siliciumdioxidsorten, insbesondere solchen, die durch Hydrolyse von SiCl₄ in einer Knallgasflamme erzeugt werden können, besonders bevorzugt aus der Gruppe der Produkte, welche die Handelsbezeichnung Aerosil^{R} tragen.

Vorteilhafte Zubereitungen betreffen Zusammensetzungen aus
(a) 5 - 90 Gew.-% eines oder mehrerer Fettalkohole, gewählt aus der Gruppe der verzweigten oder unverzweigten Alkylalkohole mit 16 - 22 C-Atomen,
(b) 1 - 90 Gew.-% eines oder mehrerer Wachsester,
(c) 0 - 20 Gew.-% hochdispersen Siliciumdioxids,
(d) gegebenenfalls einen oder mehrere kosmetische und/oder pharmazeutische Wirkstoffe enthaltend,
(e) sowie üblichen pharmazeutischen bzw. kosmetischen Hilfs- und/oder Zusatzstoffen nach Belieben (q.s.).
Bevorzugt enthalten die erfindungsgemäßen Zusammensetzungen
(a) 40 - 80 Gew.-% eines oder mehrerer Fettalkohole, gewählt aus der Gruppe der verzweigten oder unverzweigten Alkylalkohole mit 16 - 22 C-Atomen,
(b) 2 - 50 Gew.-% eines oder mehrerer Wachsester,
(c) 0,5 - 5 Gew.-% hochdispersen Siliciumdioxids,
(d) gegebenenfalls einen oder mehrere kosmetische und/oder pharmazeutische Wirkstoffe enthaltend,
(e) sowie üblichen pharmazeutischen bzw. kosmetischen Hilfs- und/oder Zusatzstoffen nach Belieben (q.s.).

Die erfindungsgemäßen Zusammensetzungen stellen vorteilhafte galenische Matrices dar, wobei der Begriff "Galenische Matrix" eine feste oder halbfeste Substanz bezeichnet, welche in der Kosmetik und/oder der Pharmazie als Trägermaterial dient. Dabei ist in den Begriff eingeschlossen, daß die Matrix als Träger für Wirkstoffe dienen kann wie auch gewünschtenfalls als Adsorptionsmittel oder Absorptionsmittel für unerwünschte, abzutransportierende Stoffe.

Die erfindungsgemäßen Zusammensetzungen können durch einfache Verfahren mit Wirkstoffen beladen werden und mit anderen vorteilhaften, ebenfalls wenig verfahrensaufwendigen Verfahren zu Mikrosphärulen verarbeitet werden.

Es ist eingangs angedeutet worden, durch welche Kennzeichen sich Mikrosphärulen auszeichnen. Im Sinne der vorliegenden Erfindung jedenfalls werden unter Mikrosphärulen feinpartikuläre Systeme, im Idealfalle kugelförmige Partikel, verstanden, bei welchen eine Trennung und Kern- und Hüllbereich nicht vorliegt. Stattdessen sollen solche Mikrosphärulen Matrixsysteme darstellen, in denen ein gegebenenfalls eingearbeiteter Wirkstoff in dispergierter oder gelöster Form inkorporiert vorliegt. Es wird, besonders wenn der Wirkstoff in fester, dispergierter Form vorliegt, nicht ausgeschlossen, daß mehrere Phasen in den Mikrosphärulen beobachtet werden können.

Die Größe der erfindungsgemäßen Mikrosphärulen beträgt vorteilhaft ca. 1 µm - 1.000 µm. Gewünschtenfalls können aus den erfindungsgemäßem Zusammensetzungen allerdings auch größere oder kleinere Partikelformate hergestellt werden. Besonders für die topische Applikation geeignete erfindungsgemäße Mikrosphärulen zeichnen sich durch Durchmesser ≦ 400 µm aus.

Erfindungsgemäß sind daher auch Mikrosphärulen zur Verwendung in kosmetischen oder pharmazeutischen Zubereitungen, dadurch gekennzeichnet, daß sie im wesentlichen zusammengesetzt sind aus
(a) einem oder mehreren Fettalkoholen, gewählt aus der Gruppe der verzweigten oder unverzweigten Alkylalkohole mit 16 - 22 C-Atomen,
(b) einem oder mehreren Wachsestern,
(c) gegebenenfalls hochdispersem Siliciumdioxid,
(d) gegebenenfalls einen oder mehrere kosmetische und/oder pharmazeutische Wirkstoffe enthaltend,
(e)( sowie gegebenenfalls übliche pharmazeutische bzw. kosmetische Hilfs- und/oder Zusatzstoffen enthaltend,
Ferner werden kosmetische oder pharmazeutische Zubereitungen, dadurch gekennzeichnet, daß sie Mikrosphärulen enthalten, welche im wesentlichen zusammengesetzt sind aus
(a) einem oder mehreren Fettalkoholen, gewählt aus der Gruppe der verzweigten oder unverzweigten Alkylalkohole mit 16 - 22 C-Atomen,
(b) einem oder mehreren Wachsestern,
(c) gegebenenfalls hochdispersem Siliciumdioxid,
(d) gegebenenfalls einen oder mehrere kosmetische und/oder pharmazeutische Wirkstoffe enthaltend,
(e) sowie gegebenenfalls übliche pharmazeutische bzw. kosmetische Hilfs- und/oder Zusatzstoffen enthaltend,
als vorteilhafte Verkörperung der hiermit vorgelegten Erfindung angesehen.

Obwohl die topische Verwendung der erfindungsgemäßen Mikrosphärulen bevorzugt ist, sind gegebenenfalls andere Darreichungsformen, beispielsweise solche zur parenteralen Ernährung oder zur peroralen Applikation, beispielsweise in Gelatinekapseln, von Vorteil.

Die erfindungsgemäßen Mikrosphärulen können auf einfache Weise nach an sich bekannten Verfahren hergestellt werden. Als besonders günstiges Herstellungsverfahren hat sich die sogenannte Sprühtrocknung bzw. Sprüherstarrung erwiesen. Es können die für solche Verfahren üblichen und gängigen Apparaturen verwendet werden, beispielsweise solche, wie sie von der Firma NIRO ATOMIZER (Söborg, Dänemark) hergestellt werden.

Als vorteilhaftes erfindungsgemäßes Herstellungsverfahren für erfindungsgemäße Mikrosphärulen wird daher ein Verfahren zur Herstellung von Mikrosphärulen angesehen, dadurch gekennzeichnet, daß eine Schmelze aus
(a) einem oder mehreren Fettalkoholen, gewählt aus der Gruppe der verzweigten oder unverzweigten Alkylalkohole mit 16 - 22 C-Atomen,
(b) einem oder mehreren Wachsestern,
(c) gegebenenfalls hochdispersem Siliciumdioxid,
(d) gegebenenfalls einen oder mehrere kosmetische und/oder pharmazeutische Wirkstoffe, welche in gelöster oder dispergierter Form vorliegen, enthaltend,
(e) sowie gegebenenfalls übliche pharmazeutische bzw. kosmetische Hilfs- und/oder Zusatzstoffen enthaltend,
mechanisch zerteilt wird und die zerteilte Schmelze zum Erstarren gebracht wird.

Die mechanische Zerteilung der Schmelze kann erfindungsgemäß vorteilhaft darin bestehen, daß die Schmelze mittels einer Düse, einer Zerstäuberscheibe oder einen Zweistoffdüsenzerstäuber zu feinen Tröpfchen zerstäubt wird.

Durch möglichst rasches Abkühlen bis zum Erstarren werden aus den so erzeugten Tröpfchen die endgültigen Mikrosphärulen erzeugt.

Es ist günstig, die Abkühlung so zu gestalten, daß die Schmelze bzw. Dispersionsschmelze in ein Kühlgas, beispielsweise Luft oder ein Inertgas, versprüht wird. Die Ableitung des mikropartikulären Sprühproduktes erfolgt vorteilhaft mit dem Kühlgasstrom, der durch einen nachgeschalteten Exhaustor aufrechterhalten werden kann.

In Zyklonen kann das Feingut abgeschieden und ausgetragen werden. Einlaßöffnungen an der Decke des Sprühturmes werden zur Kalt- oder Heißgaszufuhr genutzt, wobei die exakte Gassteuerung nach Temperatur und Menge vorteilhaft so geregelt werden kann, daß sich die Mikrosphärulen nicht in größerer Menge an der Außenwand der Sprühapparatur oder dem Zerstäuberaggregat festsetzen.

Es war überraschend und für den Fachmann nicht vorherzusehen, daß die erfindungsgemäßen kosmetischen und/oder pharmazeutischen Zusammenseztzungen die Gundlage für Mikrosphärulen darstellen würden, welche nicht nur instabile bzw. geruchlich problematische Substanzen vorzüglich zu stabilisieren vermögen, sondern sich auch durch hervorragende Freisetzungscharakteristika in Bezug auf den oder die inkorporierten Wirkstoffe auszeichnen.

Erfindungsgemäß ist auch die Verwendung von Zusammensetzungen aus
(a) einem oder mehreren Fettalkoholen, gewählt aus der Gruppe der verzweigten oder unverzweigten Alkylalkohole mit 16 - 22 C-Atomen,
(b) einem oder mehreren Wachsestern,
(c) gegebenenfalls hochdispersem Siliciumdioxid,
(d) einen oder mehrere gegen Zersetzung empfindliche kosmetische und/oder pharmazeutische Wirkstoffe enthaltend,
(e) sowie gegebenenfalls übliche pharmazeutische bzw. kosmetische Hilfs- und/oder Zusatzstoffen enthaltend,
zur Stabilisierung des oder der kosmetischen und/oder pharmazeutischen Wirkstoffes gegen Zersetzung.

Die Erfindung stellt dabei eine Kompartimentierung vom Typus fest/fest bzw. fest/halbfest bzw. flüssig/fest bzw. flüssig/halbfest bzw. halbfest/halbfest im pharmazeutischtechnologischen Sinne, dar.

Nach Einarbeiten der erfindungsgemäßen Mikrosphärulen in die erfindungemäßen Endformulierungen (z.B. Gele, Emulsionen, Hydrodispersionen) liegt ein stabiles Zweiphasensystem vor, welches die Trennung chemisch inkompatibler Substanzen in ein und demselben Präparat erlaubt und dadurch zusätzliche Stabilität schafft. Die damit erzielte Immobilisierung des Wirkstoffes oder der Wirkstoffe in den erfindungsgemäßen Zusammensetzungen bzw. Mikrosphärulen verringert zudem das Risiko möglicher unerwünschter Reaktionen mit Stoffen der umgebenden flüssigen Phase (z.B. in Gelen, Emulsionen, Lipogelen usw.).

Auch Reaktionen wie beispielsweise die Autoxidation ranziditätsgefährdeter Produkte (ungesättigte Fettsäuren, Nachtkerzenöl, Eucerit, Ceramid-Strukturen) können erfindungsgemäß unterbunden oder zumindest doch stark verzögert werden.

So wurde gefunden, daß gegen beispielsweise oxidative Zersetzungsprozesse empfindliche Substanzen, durch Inkorporierung in die erfindungsgemäßen Zusammensetzungen, vorteilhaft in Form von erfindungsgemäßen Mikrosphärulen, für einen Zeitraum, der den üblichen Haltbarkeitsdauern gewöhnlicher Kosmetika oder Pharmaka entspricht, völlig stabil sind.

Erfindungsgemäß ist daher die Verwendung von Mikrosphärulen, welche im wesentlichen zusammengesetzt sind aus
(a) einem oder mehreren Fettalkoholen, gewählt aus der Gruppe der verzweigten oder unverzweigten Alkylalkohole mit 16 - 22 C-Atomen,
(b) einem oder mehreren Wachsestern,
(c) gegebenenfalls hochdispersem Siliciumdioxid,
(d) einen oder mehrere gegen Zersetzung empfindliche kosmetische und/oder pharmazeutische Wirkstoffe enthaltend,
(e) sowie gegebenenfalls übliche pharmazeutische bzw. kosmetische Hilfs- und/oder Zusatzstoffen enthaltend,
zur Stabilisierung des oder der kosmetischen und/oder pharmazeutischen Wirkstoffes gegen Zersetzung.

Vorteilhaft und überraschend ist ferner, daß die erfindungsgemäßen galenischen Matrices nach dem Sprüherstarrungsvorgang als kugelförmige oder wenigstens annäherungsweise kugelförmige Objekte anfallen, und diese problemlos in übliche kosmetische oder pharmazeutische Grundlagen eingearbeitet werden können.

Durch den plastisch-verformbaren Charakter der Matrix wird ihre Verformbarkeit auf der Haut erleichtert und werden erfindungsgemäße Zubereitungen als besonders angenehm empfunden.

Schließlich war erstaunlich, daß als erfindungsgemäße Mikrosphärulen ausgestaltete erfindungsgemäße Zusammensetzungen sich gewünschtenfalls ohne Zusatz von Emulgatoren bereits in Wasser wie die Öltröpfchen einer O/W-Emulsion (Öl-in-Wasser-Emulsion) verhalten würden.

Dies bedeutet, daß es grundsätzlich möglich ist, aus als erfindungsgemäße Mikrosphärulen ausgestalteten erfindungsgemäßen Zusammensetzungen in Wasser, ohne weitere Zusätze, emulsionsartige Zubereitungen, welche exakt bezeichnet Dispersionen vom Typ fest/flüssig bzw. fest/halbfest im Sinne pharmazeutisch-technologischer Systeme darstellen, herzustellen.

Es ist aber auch von Vorteil, als erfindungsgemäße Mikrosphärulen ausgestaltete erfindungsgemäße Zusammensetzungen üblichen kosmetischen oder pharmazeutischen Zubereitungen, so etwa Gel-, Hydrodispersions- oder Emulsionsgrundlagen einzuverleiben. So ist es vorteilhaft, diese Mikrosphärulen in die üblichen Zubereitungen des Standes der Technik einzuarbeiten, beispielsweise in hydrophile Gele, Trägersysteme, Lipogele, Salben, Crèmes, einfache Emulsionen (der Typen W/O und O/W), multiple Emulsionen (der Typen W/O/W, O/W/O und so weiter), Mischemulsionen, Hydrodispersionen, Suspensionen und Lösungen.

Als besonders vorteilhafte Verkörperung der vorliegenden Erfindung wird angesehen, die erfindungsgemäßen Matrices in emulgatorfreie Systeme einzuarbeiten, also insbesondere in Gele, Hydrodispersionen oder Lösungen.

Die feinpartikulär vorliegenden Mikrosphärulen sind in fester, trockener Form als Zwischenprodukt ohne Zusatz weiterer Stabilisatoren wie beispielsweise Konservierungsmittel lager- und handhabbar. Ihr Einsatz ist daher völlig unproblematisch.

Sind die Zusammensetzungen gemäß der vorliegenden Erfindung kosmetischer Natur, so sind folgende Verwendungen besonders bevorzugt:

Kosmetische Gele, kosmetische Emulsionen, insbesondere vom Typ O/W sowie W/O/W, Gesichts- und Körperpflegezubereitungen aller Art, Peelingprodukte, Reinigungspräparate, pflegende Zubereitungen, den Hautzustand verbessernde oder wiederherstellende Zubereitungen, wirkstoffhaltige Zubereitungen, emulgatorfreie Zubereitungen, Sonnenschutzmittel, Insektenrepellentien, Massagegele, Sportgele.

Die Formulierung der jeweiligen kosmetischen Basis dieser Produkte ist dem Fachmanne an sich bekannt.

Handelt es sich bei den erfindungsgemäßen Zubereitungen um Emulsionen, so können die Ölbestandteile dieser Emulsionen aus allen in Kosmetik und Pharmakologie üblichen Ölen, Fetten und Wachsen gewählt werden, als da sind Paraffinöle und -wachse, pflanzliche und tierische Öle, Fette und Wachse, z.B. Hanfwachs, Flachswachs, Bienenwachs und dergleichen, Silikonöle, synthetische Wachse, z.B. Cetylpalmitat, Cetylstearat und dergleichen, Mono-, Di- und Triglyceride, Fettsäuren, Fettalkohole, Montanwachse und dergleichen mehr.

Die Wirkstoffe können sehr vorteilhaft gewählt werden aus der Gruppe der lipophilen Wirkstoffe, insbesondere aus folgender Gruppe:
Acetylsalicylsäure, Atropin, Azulen, Hydrocortison und dessen Derivaten, z.B. Hydrocortison-17-valerat, Vitamine, z.B. Ascorbinsäure und deren Derivate, Vitamine der B- und D-Reihe, sehr günstig das Vitamin B₁, das Vitamin B₁₂, das Vitamin D₃, aber auch die Vitamine A, E, insbesondere α-Tocopherylacetat, Bisabolol, ungesättigte Fettsäuren, namentlich die essentiellen Fettsäuren (oft auch Vitamin F genannt), insbesondere die gamma-Linolensäure, Ölsäure, Eicosapentaensäure, Docosahexaensäure und deren Derivate, Chloramphenicol, Coffein, Prostaglandine, Thymol, Campher, Extrakte oder andere Produkte pflanzlicher und tierischer Herkunft, z.B. Nachtkerzenöl, Borretschöl oder Johannisbeerkernöl, Fischöle, Lebertran aber auch Ceramide und ceramidähnliche Verbindungen und so weiter.

Besonders vorteilhaft ist es auch, die Wirkstoffe aus der Gruppe der rückfettenden Substanzen zu wählen, beispielsweise, Eucerit und Neocerit.

Mit dieser Auflistung ist das Potential der vorliegenden Erfindung natürlich nicht erschöpft. Vielmehr kann der Fachmann ohne erfinderisches Dazutun weitere Wirkstoffe verwenden, ohne das Gebiet der vorliegenden Erfindung zu verlassen.

Es ist erfindungsgemäß möglich und vorteilhaft, die erfindungsgemäßen Zusammensetzungen, insbesondere die erfindungsgemäßen Mikrosphärulen mit bis einem Gehalt von bis zu ca. 60 Gew.-% an Wirkstoffen zu beladen.

Es hat sich als besonders vorteilhaft erwiesen, die erfindungsgemäßen Zusammensetzungen, insbesondere die erfindungsgemäßen Mikrosphärulen mit bis einem Gehalt von 0,1 bis zu ca. 50 Gew.-% an Wirkstoffen zu beladen.

Ferner ist vorteilhaft, den Gehalt an erfindungsgemäßen Mikrosphärulen in erfindungsgemäßen kosmetischen oder pharmazeutischen Zubereitungen aus dem Bereich von 0,1 - 60 Gew.-%, insbesondere 1 - 40 Gew.-%, bezogen auf das Gesamtgewicht der Zubereitungen, zu wählen.

Für Hilfs-und Zusatzstoffe sind auch hier alle dem Fachmanne an sich bekannten Stoffe dieser Stoffgattungen möglich und vorteilhaft zu verwenden.

Bevorzugte Hilfs- und Zusatzstoffe sind beispielsweise Verdicker, Füllstoffe, Parfümöle, Farbstoffe, Wirkstoffe wie Vitamine oder Proteine, Lipidkomponenten, insbesondere Hautverwandte Lipide oder solche Lipide, die denen der Haut gleichen, Trennmittel, pharmazeutische Wirkstoffe, Lichtschutzmittel, Stabilisatoren, beispielsweise UV-Stabilisatoren, Insektenrepellentien, Alkohol, Wasser, Salze, proteolytisch oder keratolytisch wirksame Substanzen usw.

Gewünschtenfalls können auch Emulgatoren in vorteilhaften Ausführungsformen der vorliegenden Erfindungen verwendet werden.

Es ist erfindungsgemäß möglich und vorteilhaft, auf Konservierungsmittel und Antioxidantien zu verzichten. Dennoch kann die Verwendung dieser Stoffe als Hilfs- bzw. Zusatzstoff gegebenenfalls vorteilhaft sein.

Es ist weiterhin von Vorteil, die erfindungsgemäßen Zusammensetzungen mit den in der Emulsionstechnologie üblichen Mischersystemen den endgültigen Formulierungen einzuverleiben.

Die vorliegende Erfindung gestattet, lipophile Hilfs- und Wirkstoffe emulgatorfrei in der galenischen Matrix und, insbesondere, wenn die Matrix in Form von erfindungsgemäßen Mikrosphärulen vorliegt, in der jeweiligen Zubereitung zu halten, so etwa, wenn das die Mikrosphärulen umgebende System ein Gel oder eine Lösung darstellt. So ist es möglich, in diesen Systemen das hautirritative und/oder allergene Potential dieser Substanzklasse zu vermeiden. Als vorteilhafte Verkörperung emulgatorfreier erfindungsgemäßer Zubereitungen haben sich Hydrodispersionen erwiesen, welche Systeme vom Typ fest/flüssig darstellen.

Durch den partikulären Verteilungszustand in den Zubereitungen ist eine Verringerung der reaktiven Oberfläche des oder der Wirkstoffe erreichbar. Durch die Kompartimentierung ist die Reaktivität der Wirkstoffe stark vermindert.

Selbst geruchlich äußerst problematische Wirkstoffe in hohen Konzentrationen können erfindungsgemäß verarbeitet werden, ohne daß die geruchliche Akzeptanz der erfindungsgemäßen Zubereitung leiden würde.

Auch die endgültigen kosmetischen und pharmazeutischen Zubereitungen zeichnen sich durch äußerst vorteilhafte Wirkung aus. Bei ihnen zeigt sich neben der hohen Stabilität der inkorporierten Wirkstoffe wie auch der Zubereitungen selbst insbesondere die hohe Befähigung zur kontrollierten und gesteuerten Wirkstofffreisetzung nach topischer Applikation.

So hat sich gezeigt, daß die Freisetzungscharakteristiken in Hinsicht auf Freisetzungsgeschwindigkeit und Vollständigkeit der Wirkstofffreigabe kosmetischer bzw. pharmazeutischer Zubereitungen mit einem Gehalt an wirkstoffhaltigen erfindungsgemäßen Mikrosphärulen sich nicht in signifikanter Weise von solchen Zubereitungen unterscheiden, welche den gleichen Wirkstoff in einer kosmetischen bzw. pharmazeutischen Zubereitung, aber ohne eine umgebende Matrix enthalten. Dies wurde in vitro in einem Permeationsversuch an präparierter Schweinehaut ermittelt. In den erfindungsgemäßen Zubereitungen selbst diffundieren der oder die Wirkstoffe, insbesondere Coffein und α-Tocopherylacetat, nicht aus den Matrices bzw. Mikrosphärulen in das umgebende Medium hinaus.

Zu erwarten wäre gewesen, daß Matrixsysteme einen gegebenen Wirkstoff langsamer oder unvollständig freisetzen.

Ferner konnte die stabilisierende Wirkung der erfindungsgemäßen Zusammensetzungen, insbesondere der erfindungsgemäßen Mikrosphärulen anhand von Messungen im Ranzimat-Test (Firma Metrohm) überprüft werden: nach Temperaturbelastung war beispielsweise das oxidationsempfindliche Nachtkerzenöl nach wesentlich längerer Inkubationszeit die zerstört als in üblichen Formulierungen des Standes der Technik, auch im Vergleich mit solchen, in welchen das Nachtkerzenöl entweder mikroverkapselt vorlag oder durch Antioxidantien geschützt wurde. Gleiches gilt für Ceramide und ceramidähnliche Verbindungen.

Die folgenden Beispiele dienen dazu das Wesen der vorliegenden Erfindung zu erläutern, ohne daß aber beabsichtigt ist, die Erfindung auf diese Beispiele zu beschränken. Vielmehr kann der Fachmann eine Fülle von Variationen vornehmen, die seinem allgemeinen Fachwissen entspringen, die den Boden der vorliegenden Erfindung aber nicht verlassen. Die Mengenangaben der Rezepturen bzw. Beispiele bedeuten Gewichts-%, sofern nicht ausdrücklich anders gekennzeichnet.

### Rezeptur I

| | Gew.-% |
|---|---|
| Wirkstoff I (Nachtkerzenöl) | 25,00 |
| Wirkstoff II (Eucerit) | 2,50 |
| Wirkstoff III (Ceramid HO 3 von Rahn/Sederma) | 3,00 |
| Behenylalkohl | 52,00 |
| Cetylpalmitat | 8,50 |
| Ascorbylpalmitat | q.s. |
| SiO₂ hochdispers | 7,50 |

Die Rezeptur wird auf ca. 40 - 50 C erwärmt, wobei sie in eine Dispersionsschmelze übergeht. Diese Schmelze wird durch intensives Rühren homogenisiert und direkt aus dem temperierten Behälter in eine NIRO-ATOMIZER-Apparatur NIRO P7 überführt.

### Geräteparameter:

| | |
|---|---|
| Zerstäuberaggregat | Scheibe D, 14.000 Upm |
| Zulufttemperatur | 9° C |
| Ablufttemperatur | 12° C |
| Luftdurchsatz | 400 m³/h |
| Produktdurchsatz | 15,5 kg/h, Watson-Marlow Pumpe 502S, Förderleistung 25 % |

Es werden Mikrosphärulen im Größenintervall von ca. 10 - 450 µm in einer Ausbeute von > 90 %erhalten.

### Rezeptur II

| | Gew.-% |
|---|---|
| Nachtkerzenöl | 25,00 |
| Behenylalkohl | 66,00 |
| Cetylpalmitat | 7,50 |
| Propylgallat | 0,02 |
| SiO₂ hochdispers | 1,98 |

Die Rezeptur wird auf ca. 40 - 50 C erwärmt, wobei sie in eine Klarschmelze übergeht. Diese Schmelze wird durch intensives Rühren homogenisiert und direkt aus dem temperierten Behälter in eine NIRO-ATOMIZER-Apparatur NIRO P7 überführt.

### Geräteparameter:

| | |
|---|---|
| Zerstäuberaggregat | Scheibe D, 14.000 Upm |
| Zulufttemperatur | 12° C |
| Ablufttemperatur | 21° C |
| Luftdurchsatz | 400 m³/h |
| Produktdurchsatz | 15,5 kg/h, Watson-Marlow Pumpe 502S, Förderleistung 25 % |

Es werden Mikrosphärulen im Größenintervall von ca. 10 - 450 µm in einer Ausbeute von > 90 %erhalten.

### Rezeptur III

| | Gew.-% |
|---|---|
| α-Tocopherylacetat | 30,00 |
| Behenylalkohl | 65,00 |
| Cetylpalmitat | 3,50 |
| Ascorbylpalmitat | 0,50 |
| SiO₂ hochdispers | 1,00 |

Die Rezeptur wird auf ca. 40 - 50 C erwärmt, wobei sie in eine Klarschmelze übergeht. Diese Schmelze wird durch intensives Rühren homogenisiert und direkt aus dem temperierten Behälter in eine NIRO-ATOMIZER-Apparatur NIRO P7 überführt.

### Geräteparameter:

| | |
|---|---|
| Zerstäuberaggregat | Scheibe D, 14.000 Upm |
| Zulufttemperatur | 11° C |
| Ablufttemperatur | 18° C |
| Luftdurchsatz | 400 m³/h |
| Produktdurchsatz | 15,5 kg/h, Watson-Marlow Pumpe 502S, Förderleistung 25 % |

Es werden Mikrosphärulen im Größenintervall von ca. 10 - 450 µm in einer Ausbeute von > 95 %erhalten.

### Rezeptur IV

| | Gew.-% |
|---|---|
| Ceramid HO 3^{R} | 3,50 |
| Eucerit^{R} | 2,50 |
| Nachtkerzenöl | 25,00 |
| Behenylalkohl | 63,50 |
| Cetylpalmitat | 4,50 |
| Ascorbylpalmitat | 0,50 |
| SiO₂ hochdispers | 0,50 |

Die Rezeptur wird auf ca. 40 - 50 C erwärmt, wobei sie in eine Klarschmelze übergeht. Diese Schmelze wird durch intensives Rühren homogenisiert und direkt aus dem temperierten Behälter in eine NIRO-ATOMIZER-Apparatur NIRO P7 überführt.

### Geräteparameter:

| | |
|---|---|
| Zerstäuberaggregat | Scheibe D, 14.000 Upm |
| Zulufttemperatur | 9° C |
| Ablufttemperatur | 12° C |
| Luftdurchsatz | 400 m³/h |
| Produktdurchsatz | 15,5 kg/h, Watson-Marlow Pumpe 502S, Förderleistung 25 % |

Es werden Mikrosphärulen im Größenintervall von ca. 10 - 450 µm in einer Ausbeute von > 90 %erhalten.

### Rezeptur V

| | Gew.-% |
|---|---|
| Coffein | 1,50 |
| Cetylstearylalkohol | 21,00 |
| Cetylpalmitat | 76,50 |
| SiO₂ hochdispers | 1,50 |

Die Rezeptur wird auf ca. 40 - 50 C erwärmt, wobei sie in eine Dispersionsschmelze übergeht. Diese Schmelze wird durch intensives Rühren homogenisiert und direkt aus dem temperierten Behälter in eine NIRO-ATOMIZER-Apparatur NIRO P7 überführt.

### Geräteparameter:

| | |
|---|---|
| Zerstäuberaggregat | Scheibe D, 14.000 Upm |
| Zulufttemperatur | 9° C |
| Ablufttemperatur | 12° C |
| Luftdurchsatz | 400 m³/h |
| Produktdurchsatz | 15,5 kg/h, Watson-Marlow Pumpe 502S, Förderleistung 25 % |

Es werden Mikrosphärulen im Größenintervall von ca. 10 - 450 µm in einer Ausbeute von > 90 %erhalten.

In den nachfolgenden Beispielen liegen Mikrosphärulen gemäß den Rezepturen I - V vor.

### Beispiel 1

| Wäßrige Zubereitung (Gesichtswasser) | |
|---|---|
| | Gew.-% |
| PEG-40-hydrogenated Castor Oil | 0,811 |
| Dipropylenglycol | 2,534 |
| PEG-8 | 1,521 |
| Na₃EDTA | 0,253 |
| Polymer JR 125 | 0,025 |
| Rezeptur I | 0,750 |
| Wasser VES | ad 100,000 |

### Beispiel 2

| Wäßrige Zusammensetzung | |
|---|---|
| | Gew.-% |
| Polyfettsäureester (Cetiol HE) | 16,000 |
| PPG-3-Myristylether (Witconol APM) | 1,000 |
| Propylenglycol | 3,000 |
| Glycerin | 40,000 |
| Rezeptur II | 0,500 |
| Wasser VES | ad 100,000 |

### Beispiel 3

| Hydrogel (Polyacrylatgel) | |
|---|---|
| | Gew.-% |
| Acrylsäurepolymerisat (Carbopol 981) | 1,000 |
| Tris(hydroxymethylamino)methan (Tris) | 1,000 |
| Glycerin | 2,000 |
| Propylenglycol | 2,000 |
| Rezeptur III | 12,500 |
| Wasser VES | ad 100,000 |

### Beispiel 4

| Hochwasserhaltige Emulsion (sehr weich) | |
|---|---|
| | Gew.-% |
| Ceteareth (Cremophor A 25) | 0,100 |
| Cetearyl Alcohol (Lanette O) | 0,400 |
| Vaseline, DAB 9 | 12,500 |
| Mineralöl, DAB 9 | 11,000 |
| Ceteareth-6-stearylalkohol (Cremophor A6) | 6,000 |
| Rezeptur IV | 11,000 |
| Wasser VES | ad 100,000 |

### Beispiel 5

| Hochwasserhaltige Emulsion | |
|---|---|
| | Gew.-% |
| Ceteareth-25 (Cremophor A25) | 1,500 |
| Cetearyl Alcohol (Lanette O) | 8,500 |
| Rezeptur V | 8,000 |
| Wasser VES | ad 100,000 |

### Beispiel 6

| Hochwasserhaltige Emulsion | |
|---|---|
| | Gew.-% |
| Ceteareth-25 (Cremophor A25) | 2,000 |
| Cetearylalcohol (Lanette O) | 8,000 |
| Vaseline, DAB 9 | 10,000 |
| Mineralöl, DAB 9 | 10,000 |
| Rezeptur I | 10,000 |
| Wasser VES | ad 100,000 |

### Beispiel 7

| O/W-Emulsion | |
|---|---|
| | Gew.-% |
| PEG-100-Stearate (Arlacel 165) | 5,000 |
| Cetearyl Alcohol (Lanette O) | 3,000 |
| Mineralöl, DAB 9 | 25,000 |
| Paraben-Mischung | nach Belieben |
| Rezeptur III | 7,300 |
| Wasser VES | ad 100,000 |

### Beispiel 8

| O/W-Emulsion | |
|---|---|
| | Gew.-% |
| Polysorbate-60 (Tween 60) | 3,000 |
| Sorbitan Stearate (Arlacel 60) | 2,000 |
| Cetearyl Alcohol (Lanette O) | 3,000 |
| Mineralöl, DAB 9 | 25,000 |
| Paraben-Mischung | nach Belieben |
| Rezeptur IV | 0,035 |
| Wasser VES | ad 100,000 |

### Beispiel 9

| Ionische O/W-Emulsion | |
|---|---|
| | Gew.-% |
| Stearinsäure | 5,000 |
| Cetearyl Alcohol (Lanette O) | 3,000 |
| Mineralöl, DAB 9 | 25,000 |
| Paraben-Mischung | nach Belieben |
| Triethanolamin | 1,000 |
| Rezeptur IV | 17,000 |
| Wasser VES | ad 100,000 |

### Beispiel 10

| Hydrodispersionsgel mit UV-Filter | |
|---|---|
| | Gew.-% |
| Rezeptur III | 7,500 |
| 2-Phenylbenzimidazol-5-sulfonsäure, ("Eusolex 232", Merck) | 0,800 |
| Allantoin | 2,000 |
| Sorbit fl. ("Karion F", Merck) | 2,000 |
| "Carbopol 934", B.F. Goodrich | 1,500 |
| Tris (hydroxymethyl)aminomethan | 2,700 |
| Propylenglykol | 1,000 |
| Ethanol | 10,000 |
| Parfüm, Korrigentien, Additive, Antioxidantien, Stabilisatoren | q.s. |
| Wasser | ad 100,000 |

### Beispiel 11

| Tagescrème (O/W-Emulsion) mit UV-Filtern | |
|---|---|
| | Gew.-% |
| Rezeptur III | 2,500 |
| PEG-5 Glycerylstearat | 2,000 |
| Silikonöl | 1,500 |
| Eucerit^{R} | 0,500 |
| Isopropylpalmitat | 2,000 |
| C₁₂-C₁₅ Alkylbenzoat | 2,000 |
| Octyldodecanol | 1,000 |
| Butylenglycol | 2,000 |
| Cetylalkohol | 3,000 |
| Octylmethoxycinnamat | 0,500 |
| TiO₂ | 0,200 |
| Wasser VES | ad 100,000 |

Die Zubereitung wird mit einem Citratpuffer auf pH 5 gepuffert.

### Beispiel 12

| Leichte Nachtcrème (O/W-Emulsion) | |
|---|---|
| | Gew.-% |
| Rezeptur II | 7,000 |
| Hydrierte Kokosfettsäureglyceride | 5,000 |
| Octyldodecanol | 4,000 |
| Eucerit^{R} | 0,500 |
| Silikonöl | 1,000 |
| C₁₂-C₁₅-Alkylbenzoat | 4,000 |
| PEG-5 Glycerylstearat | 1,500 |
| PEG-5 Stearylstearat | 1,500 |
| Squalan | 0,500 |
| Cetylalkohol | 2,000 |
| Carbopol 934 | 0,200 |
| Schibutter | 2,000 |
| Bisabolol | 0,800 |
| Allantoin | 0,200 |
| Natriumhyaluronat | 0,100 |
| Panthenol | 1,000 |
| Wasser VES | ad 100,000 |

Die Zubereitung wird mit einem Citratpuffer auf pH 5 gepuffert.

### Beispiel 13

| | Gew.-% |
|---|---|
| Rezeptur IV | 6,500 |
| Caprylsäure/Caprinsäuretriglycerid | 2,500 |
| Paraffinöl DAB 9 | 3,500 |
| Simethicone | 0,200 |
| Isopropylpalmitat | 1,500 |
| Glycerin | 3,000 |
| Butylenglycol | 1,500 |
| Polyethylenglycol 150 | 2,000 |
| Acrylat-/C₁₀-C₃₀ Alkylacrylatcopolymer | 0,400 |
| Mg-/Al-Silicat | 0,400 |
| Polyvinylpyrrolidon | 0,500 |
| ZnSO₄ | 0,400 |
| NaOH | 0,400 |
| Ethanol | 1,000 |
| Wasser VES | ad 100,000 |

Die Zubereitung wird mit einem Citratpuffer auf pH 5 gepuffert.

## Patentansprüche

1. Zusammensetzungen aus
(a) einem oder mehreren Fettalkoholen, gewählt aus der Gruppe der verzweigten oder unverzweigten Alkylalkohole mit 16 - 22 C-Atomen,
(b) einem oder mehreren Wachsestern,
(c) gegebenenfalls hochdispersem Siliciumdioxid,
(d) gegebenenfalls einen oder mehrere kosmetische und/oder pharmazeutische Wirkstoffe enthaltend,
(e) sowie gegebenenfalls übliche pharmazeutische bzw. kosmetische Hilfs- und/oder Zusatzstoffen enthaltend.

2. Zusammensetzungen nach Anspruch 1, dadurch gekennzeichnet, daß sie
(a) 5 - 90 Gew.-% eines oder mehrerer Fettalkohole, gewählt aus der Gruppe der verzweigten oder unverzweigten Alkylalkohole mit 16 - 22 C-Atomen,
(b) 1 - 90 Gew.-% eines oder mehrerer Wachsester,
(c) 0 - 20 Gew.-% hochdispersen Siliciumdioxids,
(d) gegebenenfalls einen oder mehrere kosmetische und/oder pharmazeutische Wirkstoffe enthaltend,
(e) sowie üblichen pharmazeutischen bzw. kosmetischen Hilfs- und/oder Zusatzstoffen nach Belieben (q.s.)
enthalten.

3. Zusammensetzungen nach Anspruch 1, dadurch gekennzeichnet, daß sie
(a) 40 - 80 Gew.-% eines oder mehrerer Fettalkohole, gewählt aus der Gruppe der verzweigten oder unverzweigten Alkylalkohole mit 16 - 22 C-Atomen,
(b) 2 - 50 Gew.-% eines oder mehrerer Wachsester,
(c) 0,5 - 5 Gew.-% hochdispersen Siliciumdioxids,
(d) gegebenenfalls einen oder mehrere kosmetische und/oder pharmazeutische Wirkstoffe enthaltend,
(e) sowie üblichen pharmazeutischen bzw. kosmetischen Hilfs- und/oder Zusatzstoffen nach Belieben (q.s.),
enthalten.

4. Zusammensetzungen nach Anspruch 1, dadurch gekennzeichnet, daß der oder die Fettalkohole gewählt werden aus der Gruppe Cetylalkohol, Stearylalkohol, Cetyl-/Stearylalkohol und Behenylalkohol.

5. Zusammensetzungen nach Anspruch 1, dadurch gekennzeichnet, daß der oder die Wachsester gewählt werden aus der Gruppe der Ester höhermolekularer, bevorzugt solcher Ester, bei denen die Anzahl der C-Atome größer ist als etwa 22, und höhermolekularer Fettsäuren, bei welchen die Anzahl der C-Atome größer ist als etwa 14.

6. Mikrosphärulen zur Verwendung in kosmetischen oder pharmazeutischen Zubereitungen, dadurch gekennzeichnet, daß sie im wesentlichen zusammengesetzt sind aus
(a) einem oder mehreren Fettalkoholen, gewählt aus der Gruppe der verzweigten oder unverzweigten Alkylalkohole mit 16 - 22 C-Atomen,
(b) einem oder mehreren Wachsestern,
(c) gegebenenfalls hochdispersem Siliciumdioxid,
(d) gegebenenfalls einen oder mehrere kosmetische und/oder pharmazeutische Wirkstoffe enthaltend,
(e) sowie gegebenenfalls übliche pharmazeutische bzw. kosmetische Hilfs- und/oder Zusatzstoffen enthaltend,

7. Kosmetische oder pharmazeutische Zubereitungen, dadurch gekennzeichnet, daß sie Mikrosphärulen enthalten, welche im wesentlichen zusammengesetzt sind aus
(a) einem oder mehreren Fettalkoholen, gewählt aus der Gruppe der verzweigten oder unverzweigten Alkylalkohole mit 16 - 22 C-Atomen,
(b) einem oder mehreren Wachsestern,
(c) gegebenenfalls hochdispersem Siliciumdioxid,
(d) gegebenenfalls einen oder mehrere kosmetische und/oder pharmazeutische Wirkstoffe enthaltend,
(e) sowie gegebenenfalls übliche pharmazeutische bzw. kosmetische Hilfs- und/oder Zusatzstoffen enthaltend.

8. Verfahren zur Herstellung von Mikrosphärulen nach Anspruch 6, dadurch gekennzeichnet, daß eine Schmelze aus
(a) einem oder mehreren Fettalkoholen, gewählt aus der Gruppe der verzweigten oder unverzweigten Alkylalkohole mit 16 - 22 C-Atomen,
(b) einem oder mehreren Wachsestern,
(c) gegebenenfalls hochdispersem Siliciumdioxid,
(d) gegebenenfalls einen oder mehrere kosmetische und/oder pharmazeutische Wirkstoffe, welche in gelöster oder dispergierter Form vorliegen, enthaltend,
(e) sowie gegebenenfalls übliche pharmazeutische bzw. kosmetische Hilfs- und/oder Zusatzstoffen enthaltend,
mechanisch zerteilt wird und die zerteilte Schmelze zum Erstarren gebracht wird.

9. Verwendung von Zusammensetzungen aus
(a) einem oder mehreren Fettalkoholen, gewählt aus der Gruppe der verzweigten oder unverzweigten Alkylalkohole mit 16 - 22 C-Atomen,
(b) einem oder mehreren Wachsestern,
(c) gegebenenfalls hochdispersem Siliciumdioxid,
(d) einen oder mehrere gegen Zersetzung empfindliche kosmetische und/oder pharmazeutische Wirkstoffe enthaltend,
(e) sowie gegebenenfalls übliche pharmazeutische bzw. kosmetische Hilfs- und/oder Zusatzstoffen enthaltend,
zur Stabilisierung des oder der kosmetischen und/oder pharmazeutischen Wirkstoffes gegen Zersetzung.

10. Verwendung von Mikrosphärulen, welche im wesentlichen zusammengesetzt sind aus
(a) einem oder mehreren Fettalkoholen, gewählt aus der Gruppe der verzweigten oder unverzweigten Alkylalkohole mit 16 - 22 C-Atomen,
(b) einem oder mehreren Wachsestern,
(c) gegebenenfalls hochdispersem Siliciumdioxid,
(d) einen oder mehrere gegen Zersetzung empfindliche kosmetische und/oder pharmazeutische Wirkstoffe enthaltend,
(e) sowie gegebenenfalls übliche pharmazeutische bzw. kosmetische Hilfs- und/oder Zusatzstoffen enthaltend,
zur Stabilisierung des oder der kosmetischen und/oder pharmazeutischen Wirkstoffes gegen Zersetzung.
